# EUROPEAN PATENT SPECIFICATION

(11) **EP 1 343 454 B1**
(45) Date of publication and mention of the grant of the patent: **09.12.2009**
(21) Application number: 01990631.2
(22) Date of filing: 11.12.2001
(51) Int. Cl.: A61K 8/31, A61K 8/81, A61K 8/88, A61Q 1/10

(54) **COSMETIC COMPOSITION COMPRISING A POLYMER AND FIBRES**
KOSMETISCHE ZUSAMMENSETZUNG ENTHALTEND EIN POLYMER UND FASERN
COMPOSITION COSMETIQUE COMPRENANT UN POLYMERE ET DES FIBRES

(30) Priority: 12.12.2000 FR 0016161; 21.12.2000 WO PCT/IB00/02002
(43) Date of publication of application: 17.09.2003
(73) Proprietor: L'OREAL S.A., 75008 Paris Cedex (FR)
(72) Inventor: COLLIN, Nathalie, F-92330 Sceaux (FR)
(74) Representative: Boulard, Denis
(86) International application number: PCT/IB2001/002786
(87) International publication number: WO 2002/047619

(56) References cited:
- EP-A- 1 053 742
- EP-A- 1 066 814
- EP-A- 1 201 221
- EP-A- 1 208 836
- EP-A- 1 213 008
- FR-A- 1 529 329
- DATABASE CHEMICAL ABSTRACTS [Online] STN; access number 90: 76 419, XP002175531 & JP 53 043577 A (POLA CHEMICAL IND. CO., LTD) 21 May 1975 (1975-05-21)
- DATABASE CHEMICAL ABSTRACTS [Online] STN; access number: 124: 37403, XP002175532 & JP 07 267827 A (SHISEIDO CO LTD) 17 October 1995 (1995-10-17)
- DATABASE CHEMICAL ABSTRACTS [Online] retrieved from STN Database accession no. 96: 109968 XP002213014 & JP 56 123909 A (ISEHAN K.K.) 29 September 1981 (1981-09-29)

## Description

The present invention relates to a composition comprising, in a physiologically acceptable medium, fibres and a polymer containing a specific hetero atom, the composition being intended in particular for cosmetics. The invention also relates to a cosmetic make-up or care process for keratin materials. The make-up or care process and composition according to the invention are intended more particularly for the keratin materials of human beings, such as the skin (including the scalp), the nails, keratin fibres, especially substantially longilinear keratin fibres, such as the eyelashes, the eyebrows and the hair. The invention more especially relates to a mascara.

The composition according to the invention can be in the form of a coating composition for the eyelashes (in particular a mascara), an eyeliner, a product for the eyebrows, a product for the lips, a face powder, an eyeshadow, a foundation, a make-up product for the body, a concealer product, a nail varnish, a skincare product, including a product for scalp care, or a haircare product (hair mascara or spray).

It is known practice to use fibres in make-up products, especially for their lengthening effects in mascaras (see JP-A-57/158 714 and JP-A-3-153 613), their moisturizing properties in lipsticks (see document US-A-5 498 407), to improve the contours of lipstick on the edges of the lips (see document EP-A-0 106 762) or to improve the condition of broken nails (see FR-A-1 529 329) or in skincare products for their velvety feel (see JP-A-7/196 440). EP 1 053 742 discloses cosmetic compositions comprising fibers and polyols. However, during the application of these compositions to keratin materials, the fibres have difficulty in adhering to the keratin materials. The user must thus apply the composition to the keratin materials several times in order to deposit a sufficient amount of fibres to obtain the desired cosmetic properties, which thus requires a certain amount of time to be devoted to applying the make-up and obtaining the desired make-up results. However, users who are in a harry may find this time too long. There is thus a need to have available a composition containing fibres which allows the expected make-up result to be obtained quickly and easily.

Moreover, the fibres which do not adheres to the keratin materials thus have a tendency to become detached from their support and then become removed over time. The removal of these fibres thus causes a perceptible reduction in the desired cosmetic properties provided by the fibres, making it necessary to reapply the product. Furthermore, for a mascara, as the fibres become detached from the eyelashes, they may end up in the eyes and cause discomfort.

The aim of the present invention is to provide a cosmetic composition for overcoming the drawbacks mentioned above, comprising fibres which adhere well to keratin materials.

The Applicant has now found, surprisingly, that the use of a polymer containing a specific hetero atom in a composition comprising fibres gives a composition which is easy to apply to the keratin materials and allows the fibres to be deposited quickly on the keratin materials. The composition applied to the keratin materials allows fast and adhesive holding of the fibres on the keratin materials. The composition thus gives a deposit of the composition adhering to the keratin materials.

Furthermore, when the composition is a mascara, a make-up which quickly thickens the keratin fibres, in particular the eyelashes, its obtained. Instantaneous loading of the eyelashes is thus observed when the composition is applied thereto. The composition also gives good lengthening of the eyelashes.

More specifically, a subject of the invention is a composition comprising, in a physiologically acceptable medium containing a fatty phase, at least one first polyamide polymer with a weight-average molecular mass of less than 100 000, comprising a) a polymer, skeleton containing amide repeating units, and optionally b) at least one pendent fatty chain and/or at least one terminal fatty chain, which may be functionalized, containing from 6 to 120 carbon atoms and being linked to these amide units, and at least one or more fibres, said polymide polymer being according to claim 1.

A subject of the invention is also a cosmetic process for making up or caring for the keratin materials of human beings, comprising the application of a composition as defined above to the keratin materials.

A subject of the invention is also the use of a composition as defined above to obtain a deposit which adheres to keratin materials.

Another subject of the invention is the use of a mascara comprising a composition as defined above to thicken and/or lengthen the eyelashes.

A subject of the invention is also the use of a combination of at least one first polyamide polymer with a weight-average molecular mass of less than 100 000, comprising a) a polymer skeleton containing amide repeating units, and b) optionally at least one pendent fatty chain and/or at least one terminal fatty chain, which may be functionalized, containing from 6 to 120 carbon atoms and being linked to these amide units, and at least one fibre, in a physiologically acceptable composition, said polymide polymer being according to claim 1 to obtain a deposit which adheres to keratin materials.

The expression "physiologically acceptable medium" means a medium which is non-toxic and which can be applied to the skin, superficial body growths or the lips of human beings, such as a cosmetic medium.

For the purposes of the invention, the expression "functionalized chain" means an alkyl chain comprising one or more functional or reactive groups chosen in particular from amide, hydroxyl, ether, oxyalkylene, polyoxyalkylene and halogen groups, including fluoro or perfluoro groups, ester, siloxane and polysiloxane groups. In addition, the hydrogen atoms of one or more fatty chains may be substituted at least partially with fluorine atoms.

According to the invention, these chains may be linked directly to the polymer skeleton or via an ester function or a perfluoro group.

For the purposes of the invention, the term "polymer" means a compound containing at least 2 repeating units and preferably at least 3 repeating units, which are identical.

For the purposes of the invention, the expression "amide repeating units" means a unit containing from 2 to 80 carbon atoms and preferably from 2 to 60 carbon atoms, bearing hydrogen atoms and optionally oxygen atoms, which may be linear, branched or cyclic, and saturated or unsaturated. These units each also comprise one or more hetero atoms that are non-pendent but are in the polymer skeleton. These hetero atoms are chosen from nitrogen, optionally combined with one or more oxygen atoms. The units comprise at least one nitrogen atom, in particular a non-pendent nitrogen atom. These units also comprise a carbonyl group.

The units containing a hetero atom are amide units forming a skeleton of the polyamide type

These units are amide units. The pendent chains are advantageously linked directly to at least one of the hetero atoms of the polymer skeleton. In the invention, the first polymer comprises a polyamide skeleton.

Between the amide units, the first polymer may comprise oxyalkylene units.

In addition, the first polymer in the composition of the invention advantageously comprises a number of fatty chains which represents from 40% to 98% of the total number of units containing a hetero atom and of fatty chains, and better still from 50% to .95%. The nature and proportion of the units containing a hetero atom depends on the nature of the fatty phase and is, in particular, similar to the polar nature of the fatty phase. Thus, the more the units containing a hetero atom are polar and in high proportion in the first polymer, which corresponds to the presence of several hetero atoms, the greater the affinity of the first polymer for polar oils. On the other hand, the less polar or even apolar the units containing a hetero atom or the lower their proportion, the greater the affinity of the first polymer for apolar oils.

The first polymer is a polyamide. Thus, a subject of the invention is also a composition comprising, in a physiologically acceptable medium comprising a fatty phase, at least one first polyamide polymer with a weight-average molecular mass of less than 100 000, comprising a) a polymer skeleton containing amide repeating units, and b) optionally at least one pendent fatty chain and/or at least one terminal chain, which may be functionalized, containing from 6 to 120 carbon atoms and being linked to these amide units, and at least one fibre, said polyamide polymer being according to claim 1. The pendent fatty chains are preferably linked to at least one of the nitrogen atoms of the amide units of the first polymer.

In particular, the fatty chains of this polyamide represent from 40% to 98% of the total number of amide units and of fatty chains, and better still from 50% to 95%.

Advantageously, the first polymer, and in particular the polyamide, of the composition according to the invention has a weight-average molecular mass of less than 100 000 (especially ranging from 1 000 to 100 000), in particular less than 50 000 (especially ranging from 1 000 to 50 000) and more particularly ranging from 1 000 to 30 000, preferably from 2 000 to 20 000 and better still from 2 000 to 10 000.

The first polymer, ie the polyamide, is non soluble in water, in particular at 25 °C. In another embodiment, the first polymer has non ionic group.

As preferred first polymers which may be used in the invention, mention may be made of polyamides branched with pendent fatty chains and/or terminal fatty chains containing from 6 to 120 carbon atoms and better still from 8 to 120 and in particular from 12 to 68 carbon atoms, each terminal fatty chain being linked to the polyamide skeleton via at least one bonding group, which is an ester. These polymers comprise a fatty chain at each end of the polyamide skeleton.

These first polymers are preferably polymers resulting from a polycondensation between a dicarboxylic acid containing at least 32 carbon atoms (in particular containing from 32 to 44 carbon atoms) and at least one amine, chosen from diamines comprising at least 2 carbon atoms, (in particular from 2 to 36 carbon atoms). The diacid is preferably a dimer of a fatty acid containing ethylenic unsaturation containing at least 16 carbon atoms, preferably from 16 to 24 carbon atoms, for instance oleic acid, linoleic acid or linolenic acid. The amine can, for example, be chosen from diamine, such as ethylenediamine, hexylenediamine, hexamethylenediamine and phenylenediamine and from triamines, such as ethylenediamine. For the polymers comprising one or 2 terminal carboxylic acid groups; it is advantageous to esterify them with a monoalcohol containing at least 4 carbon atoms, preferably from 10 to 36 carbon atoms, better still from 12 to 24 and even better from 16 to 24, for example 18 carbon atoms.

These polymers are more especially those disclosed in document US-A-5 783 657 from the company Union Camp. Each of these polymers in particular satisfies formula (I) below: 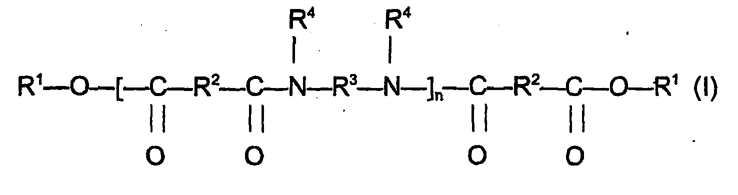 in which n denotes a number of amide units such that the number of ester groups represents from 10% to 50% of the total number of ester and amide groups; R¹ is, independently in each case, an alkyl or alkenyl group containing at least 4 carbon atoms and in particular from 4 to 24 carbon atoms; R² represents, independently in each case, a C₄ to C₄₂ hydrocarbon-based group, on condition that 50% of the groups R² represent a C₃₀ to C₄₂ hydrocarbon-based group; R³ represents, independently in each case, an organic group containing at least 2 carbon atoms, hydrogen atoms and optionally one or more oxygen or nitrogen atoms; and R⁴ represents, independently in each case, a hydrogen atom, a C₁ to C₁₀ alkyl group or a direct bond to R³ or to another R⁴, such that the nitrogen atom to which R³ and R⁴ are both attached forms part of a heterocyclic structure defined by R⁴-N-R³, with at least 50% of the groups R⁴ representing a hydrogen atom.

In the particular case of formula (I), the terminal fatty chains that are optionally functionalized for the purposes of the invention are terminal chains linked to the last hetero atom, in this case nitrogen, of the polyamide skeleton.

In particular, the ester groups of formula (I), which form part of the terminal and/or pendent fatty chains for the purposes of the invention, represent from 15% to 40% of the total number of ester and amide groups and better still from 20% to 35%. Furthermore, n is advantageously an integer ranging from 1 to 5 and better still greater than 2, i.e. from 3 to 5.

Preferably, R¹ is a C₁₂ to C₂₂ and preferably C₁₆ to C₂₂ alkyl group. Advantageously, R² can be a C₁₀ to C₄₂ hydrocarbon-based (alkylene) group. Preferably, at least 50% and better still at least 75% of the groups R² are groups containing from 30 to 42 carbon atoms. The other groups R² are C₄ to C₁₈ and better still C₄ to C₁₂ hydrogen-containing groups. Preferably, R³ represents a C₂ to C₃₆ hydrocarbon-based group or a polyoxyalkylene group and R⁴ represents a hydrogen atom. Preferably, R³ represents a C₂ to C₁₂ hydrocarbon-based group.

The hydrocarbon-based groups may be linear, cyclic or branched, and saturated or unsaturated groups. Moreover, the alkyl and alkylene groups may be linear or branched, and saturated or unsaturated groups.

In general, the polymers of formula (I) are in the form of mixtures of polymers, these mixtures also possibly containing a synthetic product corresponding to a compound of formula (I) in which n is 0, i.e. a diester.

As examples of first polymers according to the invention, mention may be made of the commercial products sold by the company Arizona Chemical under the names Uniclear^{®} 80 and Uniclear^{®} 100. They are sold, respectively, in the form of an 80% (in terms of active material) gel in a mineral oil and a 100% (in terms of active material) gel. They have a softening point of from 88 to 94°C. These commercial products are a mixture of copolymers of a C₃₆ diacid condensed with ethylenediamine, having a weight-average molecular mass of about 6 000. The terminal ester groups result from the esterification of the remaining acid endings with cetyl alcohol, stearyl alcohol or mixtures thereof (also known as cetylstearyl alcohol).

As first polymers which can be used in the invention, mention may also be made of polyamide resins resulting from the condensation of an aliphatic dicarboxylic acid and a diamine (including compounds containing more than 2 carbonyl groups and 2 amine groups), the carbonyl and amine groups of adjacent individual units being condensed via an amide bond. These polyamide resins are, in particular, those sold under the brand name Versamid^{®} by the companies General Mills Inc. and Henkel Corp. (Versamid^{®}930, 744 or 1655) or by the company Olin Mathieson Chemical Corp. under the brand name Onamid^{®}, in particular Onamid^{®} S or C. These resins have a weight-average molecular mass ranging from 6 000 to. 9 000. For further information regarding these polyamides, reference may be made to the documents US-A-3 645 705 and US-A-3 148 125. More especially, **Versamid^{®}** 930 or 744 is used.

The polyamides sold by the company Arizona Chemical under the references Uni-Rez^{®} (2658, 2931, 2970, 2621, 2613, 2624, 2665, 1554, 2623 and 2662) and the product sold under the reference Macromelt 6212 by the company Henkel may also be used. For further information regarding these polyamides, reference may be made to document US-A-5 500 209.

It is also possible to use polyamide resins obtained from plants, such as those disclosed in patents US-A-5 783 657 and US-A-5 998 570, the disclosures of which are herein incorporated by reference.

The first polymer present in the composition according to the invention advantageously has a softening point of greater than 65°C, which may be up to 190°C. It preferably has a softening point ranging from 76°c to 130°C and better still from 80°C to 105°C. The first polymer is in particular a non-waxy polymer.

The first polymer according to the invention preferably corresponds to the formula (I) mentioned above. On account of its fatty chain(s), this first polymer is readily soluble in oils and thus leads to compositions that are macroscopically homogeneous even with a high content (at least 25%) of polymer, unlike polymers not containing a fatty chain.

The first polymer may be present in the composition according to the invention in a content ranging from 0.01% to 10% by weight, relative to the total weight of the composition, preferably ranging from 0.05% to 5% by weight and better still ranging from 0.1% to 3% by weight.

According to the invention, the composition contains one or more fibres. The term "fibre" means any particle that is solid at room temperature and atmospheric pressure, whose length is greater than its apparent diameter, used alone or in combination, and which is insoluble in the ingredients of the composition, even when these ingredients are raised to a temperature above room temperature and in particular to their softening point or their melting point. These fibres are advantageously chemically inert, i.e. they do not react chemically with the various ingredients of the composition.

These fibres have melting points that are at least greater than 170°C and better still greater than 200°C. They may be absorbent or non-absorbent, i.e. capable in particular of absorbing the oils of the composition and also the biological substances secreted by the skin.

The fibres which can be used in the composition of the invention may be fibres of synthetic or natural, and inorganic or organic origin. They may be short or long, flat, cylindrical or lamellar, individual or organized for example in bundles, and hollow or solid. They can have any shape, and in particular a circular, elliptic or polygonal (triangular, square, hexagonal or octagonal) cross section depending on the specific application envisaged. In particular they can have blunt and/or rounded ends to prevent injury.

Their ends may also be multilobal, in particular trilobal, pointed or rounded.

In particular, the fibres have a length ranging from 1 nm to 20 mm, preferably from 10 nm to 5 mm and better still from 0.1 mm to 1.6 mm. Their cross section can be within a circle of diameter D ranging from 2 nm to 150 µm, preferably ranging from 20 nm to 120 µm and better still from 500 nm to 80 µm. The weight or yarn count of the fibres is often given in denier or decitex and represents the weight in grams per 9 km of yarn. The fibres in the composition according to the invention preferably have a yarn count chosen in the range from 0.15 to 30 denier and better still from 0.18 to 18 denier.

Advantageously, the fibres have a length L and a diameter D such that L/D is chosen in the range from 1.5 to 2 500, preferably from 3.5 to 500 and better still from 5 to 150.

The fibres can be those used in the manufacture of textiles, and in particular silk, cotton, wool or flax fibres, cellulose fibres extracted in particular from wood, plants or algae, polyamide (Nylon^{®}), cork, sugar can, rayon or viscose fibres, acetate fibres, in particular rayon acetate or cellulose acetate fibres, poly-(p-phenylene terephthalamide) (or aramide) fibres, in particular Kevlar^{®} fibres, acrylic polymer fibres, in particular polymethyl methacrylate (PMMA) or poly-2-hydroxyethyl methacrylate fibres, polyolefin fibres and in particular polyethylene or polypropylene fibres, glass, silica or carbon fibres, in particular in graphite form, polytetrafluoroethylene (Teflon^{®}), insoluble collagen, polyester, polyvinyl chloride or polyvinylidene chloride, polyvinyl alcohol, polyacrylonitrile, chitosan, polyurethane or polyethylene phthalate fibres, fibres formed from a mixture of polymers such as those mentioned above, for instance polyamide/polyester fibres, and mixtures thereof.

It is also possible to use surgical fibres, such as resorbable synthetic fibres prepared from glycolic acid and from e-caprolactone ("Monocryl" from Johnson & Johnson), resorbable synthetic fibres such as the copolymer of lactic acid and of glycolic acid ("Vicryl" from Johnson & Johnson), terephthalic polyester fibres ("Ethibond" from Johnson & Johnson) and stainless steel threads ("Steel" from Johnson & Johnson) in particular for use as nail varnishes.

Moreover, the fibres may or may not be surface-treated and may or may not be coated, in particular with a view to making them hydrophobic. As coated fibres which can be used in the invention, mention may be made of polyamide fibres coated with copper sulphide for an antistatic effect (for example the R-STAT fibres from Rhodia) or another polymer allowing a particular organization of the fibres (specific surface treatment) or a surface treatment which induces colour/hologram effects ("Lurex" fibre from Sildorex, for example).

Flat multilayer fibres having goniochromatic properties may also be used. Such fibres are disclosed in particular in the document. Multilayer polymer fibres are disclosed in particular in document EP-A-0 921 217. They are formed from alternating layers of polyamide and polyester, in particular polyester terephthalate.

Fibres of synthetic origin and in particular organic fibres such as those used in surgery are preferably used.

In one particular embodiment of the composition according to the invention, it is preferable for the fibre or mixture of fibres used in the composition to contain a chemical group of the same chemical nature as those of the units of the first polymer or a chemical group capable of forming physical bonds of the same type as that of the units of the first polymer (self-complementary hydrogen bonds, π interactions between unsaturated rings or charge-transfer interactions, dipolar interactions, coordination bonds with organometallic derivatives). Thus, for a first polymer containing units of the amide, urea and/or urethane type, the fibres used advantageously contain groups capable of forming hydrogen bonds, like this first polymer. As fibres capable of forming hydrogen bonds, mention may be made of fibres of acrylic polymer such as PMMA or poly(2-hydroxyethyl methacrylate), of poly- (p-phenylene terephthalamide), polyamide (Nylon^{®}) fibres, polyurethane fibres and mixtures thereof. For units of the ester type, the fibres used may be of the polyester type.

The fibres which can be used in the composition according to the invention are preferably polyamide or poly-(p-phenylene terephthalamide) fibres for a first polymer with a polyamide unit. Their length (L) can range from 0.1 to 5 mm, preferably from 0.25 to 1.6 mm, and their average diameter (D) can range from 5 to 50 µm. In particular, the polyamide fibres sold by Etablissements P. Bonte under the name Polyamide 0.9 Dtex 3 mm, having an average diameter ranging from 15 µm to 20 µm, a weight of about (0.9 dtex) and a length ranging from 0.3 mm to 1.5 mm, can be used. Poly-(p-phenylene terephthalamide) fibres with an average diameter of 12 µm and a length of about 1.5 mm can also be used, such as those sold under the name Kevlar Floc by the company Du Pont Fibres.

The fibres may be present in the composition according to the invention in a content ranging from 0.1% to 40% by weight, relative to the total weight of the composition, in particular ranging from 0.5% to 30% by weight, preferably ranging from 1% to 20% by weight and better still from 1% to 10% by weight.

Advantageously, the first polymer and the fibres may be present in the composition according to the invention in a fibres/first polymer weight ratio which may range from 0.5 to 4, preferably ranging from 0.9 to 2.5.

The fatty phase of the composition can comprise fatty substances chosen from oils, organic solvents, waxes and pasty fatty substances, and mixtures thereof. The fatty phase can form a continuous phase of the composition. In particular, the composition according to the invention may be anhydrous.

The fatty phase may especially consist of any oil which is physiologically acceptable and in particular cosmetically acceptable, chosen especially from oils of mineral, animal, plant or synthetic origin, carbon-based oils, hydrocarbon-based oils, fluoro oils and/or silicone oils, alone or as a mixture, provided that they form a homogeneous and stable mixture and provided that they are compatible with the intended use.

The total fatty phase of the composition can represent from 2% to 98% by weight, relative to the total weight of the composition, and preferably from 5% to 85% by weight.

The fatty phase of the composition can advantageously comprise at least one volatile oil or organic solvent and/or at least one non-volatile oil.

For the purposes of the invention, the expression "volatile oil or organic solvent" means any non-aqueous medium which can evaporate on contact with the skin in less than one hour at room temperature and atmospheric pressure. The volatile organic solvent(s) and the 'volatile oils of the invention are volatile cosmetic organic solvents and oils, that are liquid at room temperature, having a non-zero vapour pressure at room temperature and atmospheric pressure, ranging in particular from 10⁻³ to 300 mmHg (0.13 Pa to 40 000 Pa) and preferably greater than 0.3 mmHg (30 Pa). The expression "non-volatile oil" means an oil which remains on the skin at room temperature and atmospheric pressure for at least several hours and which in particular has a vapour pressure of less than 10⁻² mmHg (1.33 Pa).

These oils may be hydrocarbon-based oils, silicone oils or fluoro oils, or mixtures thereof.

The expression "hydrocarbon-based oil" means an oil mainly containing hydrogen and carbon atoms and optionally oxygen, nitrogen, sulphur or phosphorus atoms. The volatile hydrocarbon-based oils may be chosen from hydrocarbon-based oils containing from 8 to 16 carbon atoms, and especially C₈-C₁₆ branched alkanes, for instance C₈-C₁₆ isoalkanes of petroleum origin (also known as isoparaffins), for instance isododecane (also known as 2,2,4,4,6-pentamethylheptane), isodecane and isohexadecane, and, for example, the oils sold under the trade names Isopars or Permetyls, C₈-C₁₆ branched esters, isohexyl neopentanoate, and mixtures thereof. Other volatile hydrocarbon-based oils, for instance petroleum distillates, especially those sold under the name Shell Solt by the company Shell, may also be used. The volatile solvent is preferably chosen from hydrocarbon-based volatile oils containing from 8 to 16 carbon atoms, and mixtures thereof.

Volatile oils which may also be used are volatile silicones such as, for example, linear or cyclic volatile silicone oils, especially those with a viscosity ≤ 8 centistokes (8 × 10⁻⁶ m²/s) and especially containing from 2 to 7 silicon atoms, these silicones optionally comprising alkyl or alkoxy groups containing from 1 to 10 carbon atoms. As volatile silicone oils which may be used in the invention, mention may be made in particular of octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane and dodecamethylpentasiloxane, and mixtures thereof.

Volatile fluoro solvents such as nonafluoromethoxybutane or perfluoromethylcyclopentane may also be used.

The volatile oil may be present in the composition according to the invention in a content ranging from 0% to 98% by weight (in particular from 0.1% to 98%), relative to the total weight of the composition, preferably from 0% to 65% by weight (in particular from 1% to 65%).

The composition can also comprise at least one non-volatile oil chosen in particular from non-volatile hydrocarbon-based and/or silicone and/or fluoro oils.

Non-volatile hydrocarbon-based oils which may be mentioned in particular are:
- hydrocarbon-based plant oils such as triglycerides consisting of fatty acid esters and of glycerol in which the fatty acids may have varied chain lengths from C₄ to C₂₄, these chains possibly being linear or branched, and saturated or unsaturated; these oils are, in particular, wheat germ oil, sunflower oil, grape seed oil, sesame oil, corn oil, apricot oil, castor oil, karite butter, avocado oil, olive oil, soybean oil, sweet almond oil, palm oil, rape seed oil, cotton oil, hazelnut oil, macadamia oil, jojoba oil, alfalfa oil, poppy oil, pumpkin oil, marrow oil, blackcurrant seed oil, evening primrose oil, millet oil, barley oil, quinoa oil, rye oil, safflower oil, candlenut oil, passion flower oil and musk rose oil; or alternatively caprylic/capric acid triglycerides such as those sold by Stearineries Dubois or those sold under the names Miglyol 810, 812 and 818 by Dynamit Nobel;
- synthetic ethers containing from 10 to 40 carbon atoms;
- linear or branched hydrocarbons of mineral or synthetic origin, such as petroleum jelly, polydecenes, hydrogenated polyisobutene such as parleam, and squalane, and mixtures thereof;
- synthetic esters such as oils of formula R₁COOR₂ in which R₁ represents a linear or branched fatty acid residue containing from 1 to 40 carbon atoms and R₂ represents an in particular branched hydrocarbon-based chain containing from 1 to 40 carbon atoms, on condition that R₅ + R₆ 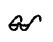 10, such as, for example, purcellin oil (cetostearyl octanoate), isopropyl myristate, isopropyl palmitate, C₁₂-C₁₅ alkyl benzoate, hexyl laurate, diisopropyl adipate, isononyl isononanoate, 2-ethylhexyl palmitate, isostearyl isostearate, alkyl or polyalkyl octanoates, decanoates or ricinoleates such as propylene glycol dioctanoate; hydroxylated esters such as isostearyl lactate and diisostearyl malate; and pentaerythritol esters;
- fatty alcohols that are liquid at room temperature, containing a branched and/or unsaturated carbon-based chain containing from 12 to 26 carbon atoms, for instance octyldodecanol, isostearyl alcohol, oleyl alcohol, 2-hexyldecanol, 2-butyloctanol or 2-undecylpentadecanol;
- higher fatty acids such as oleic acid, linoleic acid or linolenic acid;
and mixtures thereof.

The non-volatile silicone oils which may be used in the composition according to the invention may be non-volatile polydimethylsiloxanes (PDMSs), polydimethylsiloxanes comprising alkyl or alkoxy groups, that are pendent and/or at the end of a silicone chain, the groups each containing from 2 to 24 carbon atoms, phenylsilicones, for instance phenyltrimethicones, phenyldimethicones, phenyl-trimethylsiloxydiphenylsiloxanes, diphenyldimethicones, diphenylmethyldiphenyltrisiloxanes and 2-phenylethyl trimethylsiloxysilicates.

The fluoro oils which may be used in the invention are, in particular, fluorosilicone oils, fluoropolyethers or fluorosilicones, as described in document EP-A-847 752.

The non-volatile oils may be present in the composition according to the invention in a content ranging from 0% to 80% (in particular from 0.1% to 80%) by weight, preferably from 0% to 50% by weight (in particular 0.1% to 50% by weight), relative to the total weight of the composition, and better still from 0% to 20% by weight (in particular 0.1% to 20%).

The fatty phase of the composition according to the invention can comprise a wax. For the purposes of the present invention, the term "wax" means a lipophilic fatty compound that is solid at room temperature (25°C) and atmospheric pressure (760 mmHg, i.e. 10⁵ Pa), which undergoes a reversible solid/liquid change of state and which has a melting point of greater than 30°C and better still greater than 55°C, which may be up to 200°C, in particular up to 120°C.

By taking the wax to its melting point, it is possible to make it miscible with oils and to form a microscopically homogeneous mixture, but on returning the temperature of the mixture to room temperature, recrystallization of the wax in the mixture of oils is obtained.

According to the invention, the melting point values correspond to the melting peak measured using a differential scanning calorimeter (DSC), for example the calorimeter sold under the name DSC 30 by the company Mettler, with a temperature increase of 5 or 10°C per minute.

For the purposes of the invention, the waxes are those generally used in cosmetics and dermatology. Mention may be made in particular of beeswax, lanolin wax, Chinese insect waxes, rice wax, carnauba wax, candelilla wax, ouricury wax, sugar cane wax, Japan wax, sumach wax, montan wax, microcrystalline waxes, paraffin waxes, ozokerites, ceresin wax, lignite wax, polyethylene waxes and the waxes obtained by Fisher-Tropsch synthesis, and fatty acid esters of glycerides that are solid at 40°C and better still at more than 55°C. Mention may also be made of the waxes obtained by catalytic hydrogenation of animal or plant oils containing linear or branched C₈-C₃₂ fatty chains. Among these, mention may be made in particular of hydrogenated jojoba oil, hydrogenated sunflower oil, hydrogenated castor oil, hydrogenated coconut oil and hydrogenated lanolin oil.

Mention may also be made of silicone waxes or fluoro waxes.

The waxes present in the composition may be dispersed in the form of particles in an aqueous medium. These particles may have an average size ranging from 50 nm to 10 µm and preferably from 50 nm to 3.5 µm. In particular, the wax may be present in the form of a wax-in-water emulsion, the waxes possibly being in the form of particles with an average size ranging from 1 µm to 10 µm and preferably from 1 µm to 3.5 µm.

In another embodiment of the composition according to the invention, the wax may be present in the form of a wax microdispersion, the wax being in the form of particles with an average size of less than 1 µm and in particular ranging from 50 nm to 500 nm. Wax microdispersions are disclosed in documents EP-A-557. 196 and EP-A-1 048 282.

The wax may also have a hardness ranging from 0.05 MPa to 15 MPa and preferably ranging from 6 MPa to 15 MPa. The hardness is determined by measuring the compressive strength, measured at 20°C using a texturometer sold under the name TA-XT2i by the company Rheo, equipped with a stainless steel cylinder 2 mm in diameter travelling at a measuring speed of 0.1 mm/s, and penetrating into the wax to a penetration depth of 0.3 mm. To carry out the hardness measurement, the wax is melted at a temperature equal to the melting point of the wax +20°C. The molten wax is cast in a container 30 mm in diameter and 20 mm deep. The wax is recrystallized at room temperature (25°C) over 24 hours and is then stored for at least one hour at 20°C before carrying out the hardness measurement. The value of the hardness is the compressive strength measured divided by the area of the texturometer cylinder in contact with the wax.

The wax may be present in the composition according to the invention in a content ranging from 0.1% to 50% by weight, relative to the total weight of the composition, preferably from 0.5% to 30% by weight and better still from 1% to 20% by weight.

The composition according to the invention may contain at least one fatty compound that is pasty at room temperature. For the purposes of the invention, the expression "pasty fatty substance" means fatty substances with a melting point ranging from 20 to 55°C, preferably 25 to 45°C, and/or a viscosity at 40°C ranging from 0.1 to 40 Pa.s (1 to 400 poises), preferably 0.5 to 25 Pa.s, measured using a Contraves TV or Rheomat 80 viscometer, equipped with a spindle rotating at 60 Hz. A person skilled in the art can select the spindle for measuring the viscosity from the spindles MS-r3 and MS-r4, on the basis of his general knowledge, so as to be able to carry out the measurement of the pasty compound tested.

These fatty substances are preferably hydrocarbon-based compounds, optionally of polymeric type; they can also be chosen from silicone compounds and/or fluoro compounds; they may also be in the form of a mixture of hydrocarbon-based compounds and/or silicone compounds and/or fluoro compounds. In the case of a mixture of different pasty fatty substances, the hydrocarbon-based pasty compounds (containing mainly hydrogen and carbon atoms and optionally ester groups) are preferably used in major proportion.

Among the pasty compounds which may be used in the composition according to the invention, mention may be made of lanolins and lanolin derivatives such as acetylated lanolins or oxypropylenated lanolins or isopropyl lanolate, having a viscosity of from 18 to 21 Pa.s, preferably 19 to 20.5 Pa.s, and/or a melting point of from 30 to 55°C, and mixtures thereof. It is also possible to use esters of fatty acids or of fatty alcohols, in particular those containing from 20 to 65 carbon atoms. (melting point of about from 20 to 35°C and/or viscosity at 40°C ranging from 0.1 to 40 Pa.s), such as triisostearyl or cetyl citrate; arachidyl propionate; polyvinyl laurate; cholesterol esters, such as triglycerides of plant origin, such as hydrogenated plant oils, viscous polyesters such as poly(12-hydroxystearic acid), and mixtures thereof. Triglycerides of plant origin which may be used are hydrogenated castor oil derivatives, such as " Thixinr" from Rhéox.

Mention may also be made of pasty silicone fatty substances such as polydimethylsiloxanes (PDMSs) containing pendent chains of the alkyl or alkoxy type containing from 8 to 24 carbon atoms, and having a melting point of 20-55°C, such as stearyldimethicones, in particular those sold by Dow Corning under the trade names DC2503 and DC25514, and mixtures thereof.

The pasty fatty substance may be present, in the composition according to the invention in a proportion of from 0% to 60% (in particular 0.01% to 60%) by weight, relative to the total weight of the composition, preferably in a proportion of from 0.5% to 45 % by weight, and better still ranging from 2% to 30% by weight, in the composition.

The composition according to the invention may also comprise an aqueous medium, constituting an aqueous phase, which may be the continuous phase of the composition.

The aqueous phase may consist essentially of water; it may also comprise a mixture of water and of water-miscible solvent (miscibility in water of grater than 50% by weight at 25°C), for instance lower monoalcohols containing from 1, to 5 carbon atoms such as ethanol or, isopropanol, glycols containing from 2 to 8 carbon atoms, such as propylene glycol, ethylene glycol, 1,3-butylene glycol or dipropylene glycol, C₃-C₄ ketones and C₂-C₄ aldehydes.

The aqueous phase (water and optionally the water-miscible organic solvent) may be present in a content ranging from 1% to 95% by weight, relative to the total weight of the composition, preferably from 5% to 80% by weight and better still from 10% to 60% by weight.

The composition according to the invention can contain emulsifying surfactants, present in particular in a proportion ranging from 2% to 30% by weight relative to the total weight of the composition, and better still from 5% to 15%. These surfactants may be chosen from anionic and nonionic surfactants. Reference may be made to the document encyclopedia of Chemical Technology, Kirk-Othmer", volume 22, pp. 333-432, 3rd edition, 1979, Wiley, for the definition of the properties and functions (emulsifying) of surfactants, in particular pp. 347-377 of the said reference, for the anionic and nonionic surfactants.

The surfactants preferably used in the composition according to the invention are chosen from:
- nonionic surfactants: fatty acids, fatty alcohols, polyethoxylated or polyglycerolated fatty alcohols such as polyethoxylated stearyl or cetylstearyl alcohol, fatty acid esters of sucrose, alkylglucose esters, in particular polyoxyethylenated fatty esters of C₁-C₆ alkyl glucose, and mixtures thereof;
- anionic surfactants: C₁₆-C₃₀ fatty acids neutralized with amines, aqueous ammonia or alkaline salts, and mixtures thereof.

Surfactants which make it possible to obtain an oil-in-water or wax-in-water emulsion are preferably used.

The composition according to the invention can comprise at least one additional second film-forming polymer, different from the first polymer described above.

The second film-forming polymer may be a polymer which is dissolved or dispersed in the form of particles in an aqueous phase of the composition, or dissolved or dispersed in the form of particles in a liquid fatty phase. The composition can comprise a mixture of these polymers.

The second film-forming polymer may be present in the composition according to the invention in a solids content ranging from 0.1% to 60% by weight relative to the total weight of the composition, preferably from 0.5% to 40% by weight and better still from 1% to 30% by weight.

In the present application, the expression "film-forming polymer" means a polymer which is capable, by itself or in the presence of an auxiliary film-forming agent, of forming a continuous and adherent film on a support, in particular on keratin materials.

A film-forming polymer capable of forming a hydrophobic film, i.e. a polymer whose film has a water-solubility at 25°C of less than 1% by weight, is preferably used.

Among the film-forming polymers which may be used in the composition of the present invention, mention may be made of synthetic polymers, of radical-mediated type or of polycondensate type, and polymers of natural origin, and mixtures thereof.

The expression "radical-mediated film-forming polymer" means a polymer obtained by polymerization of monomers containing unsaturation, in particular ethylenic unsaturation, each monomer being capable of homopolymerizing (unlike polycondensates).

The film-forming polymers of radical-mediated type may be, in particular, vinyl polymers or copolymers, in particular acrylic polymers.

The vinyl film-forming polymers can result from the polymerization of monomers containing ethylenic unsaturation and containing at least one acidic group and/or esters of these acidic monomers and/or amides of these acidic monomers.

Monomers bearing an acidic group which may be used are α,β-ethylenic unsaturated carboxylic acids such as acrylic acid, methacrylic acid, crotonic acid, maleic acid or itaconic acid. (Meth)acrylic acid and crotonic acid are preferably used, and more preferably (meth)acrylic acid.

The esters of.acidic monomers are advantageously chosen from (meth)acrylic acid esters (also known as (meth)acrylates), especially (meth)acrylates of an alkyl, in particular of a C₁-C₃₀ and preferably C₁-C₂₀ alkyl, (meth)acrylates of an aryl, in particular of a C₆-C₁₀ aryl, and (meth) acrylates of a hydroxyalkyl, in particular of a C₂-C₆ hydroxyalkyl.

Among the alkyl (meth)acrylates which may be mentioned are methyl methacrylate, ethyl methacrylate, butyl methacrylate, isobutyl methacrylate, 2-ethylhexyl methacrylate, lauryl methacrylate and cyclohexyl methacrylate.

Among the hydroxyalkyl (meth)acrylates which may be mentioned are hydroxyethyl acrylate, 2-hydroxypropyl acrylate, hydroxyethyl methacrylate and 2-hydroxypropyl methacrylate.

Among the aryl (meth)acrylates which may be mentioned are benzyl acrylate and phenyl acrylate.

The (meth)acrylic acid esters that are particularly preferred are the alkyl (meth)acrylates.

According to the present invention, the alkyl group of the esters may be either fluorinated or perfluorinated, i.e. some or all of the hydrogen atoms of the alkyl group are substituted with fluorine atoms.

As amides of the acidic monomers, mention may be made, for example, of (meth)acrylamides, and especially N-alkyl(meth)acrylamides, in particular of a C₂-C₁₂ alkyl. Among the N-alkyl(meth)acrylamides which may be mentioned are N-ethylacrylamide, N-t-butylacrylamide, N-t-octylacrylamide and N-undecylacrylamide.

The vinyl film-forming polymers can also result from the homopolymerization or copolymerization of monomers chosen from vinyl esters and styrene monomers. In particular, these monomers may be polymerized with acidic monomers and/or esters thereof and/or amides thereof, such as those mentioned above.

Examples of vinyl esters which may be mentioned are vinyl acetate, vinyl neodecanoate, vinyl pivalate, vinyl benzoate and vinyl t-butylbenzoate.

Styrene monomers which may be mentioned are styrene and α-methylstyrene.

It is possible to use any monomer known to those skilled in the art which falls within the categories of acrylic and vinyl monomers (including monomers modified with a silicone chain).

Among the film-forming polycondensates which may be mentioned are polyurethanes, polyesters, polyesteramides, polyamides, epoxy ester resins and polyureas.

The polyurethanes may be chosen from anionic, cationic, nonionic and amphoteric polyurethanes, polyurethane-acrylics, polyurethane-polyvinylpyrrolidones, polyester-polyurethanes, polyether-polyurethanes, polyureas and polyurea-polyurethanes, and mixtures thereof.

The polyesters may be obtained, in a known manner, by polycondensation of dicarboxylic acids with polyols, in particular diols.

The dicarboxylic acid may be aliphatic, alicyclic or aromatic. Examples of such acids which may be mentioned are: oxalic acid, malonic acid, dimethylmalonic acid, succinic acid, glutaric acid, adipic acid, pimelic acid, 2,2-dimethylglutaric acid, azelaic acid, suberic acid, sebacic acid, fumaric acid, maleic acid, itaconic acid, phthalic acid, dodecanedioic acid, 1,3-cyclohexanedicarboxylic acid, 1,4-cyclohexanedicarboxylic acid, isophthalic acid, terephthalic acid, 2,5-norboranedicarboxylic acid, diglycolic acid, thiodipropionic acid, 2,5-naphthalenedicarboxylic acid and 2,6-naphthalenedicarboxylic acid. These dicarboxylic acid monomers may be used alone or in combination with at least two dicarboxylic acid monomers. Among these monomers, the ones preferably chosen are phthalic acid, isophthalic acid and terephthalic acid.

The diol may be chosen from aliphatic, alicyclic and aromatic diols. The diol preferably used is one chosen from: ethylene glycol, diethylene glycol, triethylene glycol, 1,3-propanediol, cyclohexanedimethanol, 4-butanediol. Other polyols which may be used are glycerol, pentaerythritol, sorbitol and trimethylolpropane.

The polyesteramides may be obtained in a manner analogous to that of the polyesters, by polycondensation of diacids with diamines or amino alcohols. Diamines which may be used are ethylenediamine, hexamethylenediamine and meta- or para-phenylenediamine. An amino alcohol which may be used is monoethanolamine.

The polyester may also comprise at least one monomer bearing at least one group -SO₃M, with M representing a hydrogen atom, an ammonium ion NH₄+ or a metal ion such as, for example, an Na⁺, Li⁺, K⁺, Mg²⁺, Ca²⁺, Cu²⁺, Fe²⁺ or Fe³⁺ ion. A difunctional aromatic monomer comprising such a group -SO₃M may be used in particular.

The aromatic nucleus of the difunctional aromatic monomer also bearing a group -SO₃M, as described above may be chosen, for example, from benzene, naphthalene, anthracene, biphenyl, oxybiphenyl, sulphonylbiphenyl and methylenebiphenyl nuclei. As examples of difunctional aromatic monomers also bearing a group -SO₃M, mention may be made of: sulphoisophthalic acid, sulphoterephthalic acid, sulphophthalic acid, 4-sulphonaphthalene-2,7-dicarboxylic acid.

The copolymers preferably used are those based on isophthalate/sulphoisophthalate, and more particularly copolymers obtained by condensation of diethylene glycol, cyclohexanedimethanol, isophthalic acid and sulphoisophthalic acid. Such polymers are sold, for example, under the brand name Eastman AQ^{®} by the company Eastman Chemical Products.

The polymers of natural origin, optionally modified, may be chosen from shellac resin, sandarac gum, dammar resins, elemi gums, copal resins and cellulose polymers, and mixtures thereof.

According to a first embodiment of the composition according to the invention, the second film-forming polymer may be present in the form of particles in aqueous dispersion, which is generally known as a latex or pseudolatex. The techniques for preparing these dispersions are well known to those skilled in the art.

Aqueous dispersions of film-forming polymers which may be used are the acrylic dispersions sold under the names Neocryl XK-90^{®}, Neocryl A-1070^{®}, Neocryl A-1090^{®}, Neocryl BT-62^{®}, Neocryl A-1079^{®} and Neocryl A-523^{®} by the company Avecia-Neoresins, Dow Latex 432^{®} by the company Dow Chemical, Daitosol 5000 AD^{®} by the company Daito Kasey Kogyo; or the aqueous dispersions of polyurethane sold under the names Neorez R-981^{®} and Neorez R-974^{®} by the company Avecia-Neoresins, Avalure UR-405^{®}, Avalure UR-410^{®}, Avalure UR-425^{®}, Avalure UR-450^{®}, Sancure 875^{®}, Sancure 861^{®}, Sancure 878^{®} and Sancure 2060^{®} by the company Goodrich, Impranil 85^{®} by the company Bayer and Aquamere H-1511^{®} by the company Hydromer.

Aqueous dispersions of film-forming polymers which may also be used are the polymer dispersions resulting from the radical-mediated polymerization of one or more radical-mediated monomers within and/or partially at the surface of pre-existing particles of at least one polymer chosen from the group consisting of polyurethanes, polyureas, polyesters, polyesteramides and/or alkyds. These polymers are generally referred to as hybrid polymers.

According to a second embodiment of the composition according to the invention, the film-forming polymer may be a water-soluble polymer and is thus present in the aqueous phase of the composition in dissolved form.

Examples of water-soluble film-forming polymers which may be mentioned are:
- proteins, for instance proteins of plant origin such as wheat proteins and soybean proteins; proteins of animal origin such as keratins, for example keratin hydrolysates and sulphonic keratins;
- anionic, cationic, amphoteric or nonionic chitin or chitosan polymers;
- polymers of celluloses such as hydroxyethylcellulose, hydroxypropylcellulose, methylcellulose, ethylhydroxyethylcellulose and carboxymethylcellulose, and quaternized cellulose derivatives;
- acrylic polymers or copolymers, such as polyacrylates or polymethacrylates;
- vinyl polymers, for instance polyvinylpyrrolidones, copolymers of methyl vinyl ether and of malic anhydride, the copolymer of vinyl acetate and of crotonic acid, copolymers of vinylpyrrolidone and of vinyl acetate; copolymers of vinylpyrrolidone and of caprolactam; polyvinyl alcohol;
- polymers, of natural origin, which are optionally modified, such as:
- gum arabics, guar gum, xanthan derivatives, karaya gum;
- alginates and carrageenans;
- glycoaminoglycans, hyaluronic acid and derivatives thereof;
- shellac resin, sandarac gum, dammar resins, elemi gums and copal resins;
- deoxyribonucleic acid;
- mucopolysaccharides such as hyaluronic acid and chondroitin sulphate, and mixtures thereof.

According to another embodiment of the composition according to the invention, the film-forming polymer may be present in a liquid fatty phase comprising organic solvents or oils such as those described above. For the purposes of the invention, the expression "liquid fatty phase" means a fatty phase which is liquid at room temperature (25°C) and .atmospheric pressure (760 mmHg, i.e. 10⁵ Pa), composed of one or more fatty substances that are liquid at room temperature, also known as oils, which are generally mutually compatible.

The liquid fatty phase preferably comprises a volatile oil, optionally mixed with a non-volatile oil, the oils possibly being chosen from those mentioned above.

According to a third embodiment of the composition according to the invention, the film-forming polymer may be present in the form of surface-stabilized particles dispersed in the liquid fatty phase.

The dispersion of surface-stabilized polymer particles may be manufactured as disclosed in document EP-A-749 747.

The polymer particles are surface-stabilized by means of a stabilizer which may be a block polymer, a grafted polymer and/or a random polymer, alone or as a mixture.

Dispersions of film-forming polymer in the liquid fatty phase, in the presence of stabilizers, are disclosed in particular, in documents EP-A-0 749 746, EP-A-0 923 928 and EP-A-0 930 060, the content of which is incorporated in the present patent application by reference.

The size of the polymer particles dispersed either in the aqueous phase or in the liquid fatty phase can range from 5 nm to 600 nm and preferably from 20 nm to 300 nm.

According to a fourth embodiment of the composition according to the invention, the film-forming polymer may be dissolved in the liquid fatty phase, in which case the film-forming polymer is said to be a liposoluble polymer.

Examples of liposoluble polymers which may be mentioned are copolymers of vinyl ester (the vinyl group being directly linked to the oxygen atom of the ester group and the vinyl ester containing a saturated, linear or branched hydrocarbon-based radical of 1 to 19 carbon atoms, linked to the carbonyl of the ester group) and of at least one other monomer which may be a vinyl ester (other than the vinyl ester already present), an α-olefin (containing from 8 to 28 carbon atoms), an alkyl vinyl ether (in which the alkyl group comprises from 2 to 18 carbon atoms) or an allylic or methallylic ester (containing a saturated, linear or branched hydrocarbon-based radical of 1 to 19 carbon atoms, linked to the carbonyl of the ester group).

These copolymers may be crosslinked with the aid of crosslinking agents, the aim of which is to [lacuna] which may be either of the vinyl type or of the allylic or methallylic type, such as tetraallyloxyethane, divinylbenzene, divinyl octanedioate, divinyl dodecanedioate and divinyl octadecanedioate.

Examples of these copolymers which may be mentioned are the following copolymers: vinyl acetate/ allyl stearate, vinyl acetate/vinyl laurate, vinyl acetate/vinyl stearate, vinyl acetate/octadecene, vinyl acetate/octadecyl vinyl ether, vinyl propionate/allyl laurate, vinyl propionate/vinyl- laurate, vinyl stearate/1-octadecene, vinyl acetate/1-dodecene, vinyl stearate/ethyl vinyl ether, vinyl propionate/cetyl vinyl ether, vinyl stearate/allyl acetate, vinyl 2,2-dimethyloctanoate/vinyl laurate, allyl 2,2-dimethylpentanoate/vinyl laurate, vinyl dimethylpropionate/vinyl stearate, allyl dimethylpropionate/vinyl stearate, vinyl propionate/ vinyl stearate, crosslinked with 0.2% divinylbenzene, vinyl dimethylpropionate/vinyl laurate, crosslinked with 0.2% divinylbenzene, vinyl acetate/octadecyl vinyl ether, crosslinked with 0.2% tetaallyloxyethane, vinyl acetate/allyl stearate, crosslinked with 0.2% divinylbenzene, vinyl acetate/1-octadecene, crosslinked with 0.2% divinylbenzene, and allyl propionate/allyl stearate, crosslinked with 0.2% divinylbenzene.

Examples of liposoluble film-forming polymers which may also be mentioned are liposoluble homopolymers, and in particular those resulting from the homopolymerization of vinyl esters containing from 9 to 22 carbon atoms or of alkyl acrylates or methacrylates, and alkyl radicals containing from 10 to 20 carbon atoms.

Such liposoluble homopolymers may be chosen from polyvinyl stearate, polyvinyl stearate crosslinked with divinylbenzene, with diallyl ether or with diallyl phthalate, polystearyl (meth)acrylate, polyvinyl laurate and polylauryl (meth) acrylate, it being possible for these poly(meth)acrylates to be crosslinked with the aid of ethylene glycol dimethacrylate or tetraethylene glycol dimethacrylate.

The liposoluble copolymers and homopolymers defined above are known and are described in particular in patent application FR-A-2 232 303; they may have a weight-average molecular weight ranging from 2 000 to 500 000 and preferably from 4 000 to.200 000.

As liposoluble film-forming polymers which may be used in the invention, mention may also be made of polyalkylenes and in particular copolymers of C₂-C₂₀ alkenes, such as polybutene, alkylcelluloses with a linear or branched, saturated or unsaturated C₁-C₈ alkyl radical, for instance ethylcellulose and propylcellulose, copolymers of -vinylpyrrolidone (VP) and in particular copolymers of vinylpyrrolidone and of C₂ to C₄₀ and better still C₃ to C₂₀ alkene. As examples of VP copolymers which may be used in the invention, mention may be made of the copolymers of VP/vinyl acetate, VP/ethyl methacrylate, butylated polyvinylpyrrolidone (PVP), VP/ethyl methacrylate/ methacrylic acid, VP/eicosene, VP/hexadecene, VP/triacontene, VP/styrene or VP/acrylic acid/lauryl methacrylate.

The composition according to the invention may comprise an auxiliary film-forming agent for promoting the formation of a film with the film-forming polymer. Such a film-forming agent may be chosen from any compound known to those skilled in the art as being capable of fulfilling the desired function, and may be chosen in particular from plasticizers and coalescers.

The composition according to the invention may also comprise a dyestuff, for instance pulverulent dyestuffs, liposoluble dyes and water-soluble dyes. This dyestuff may be present in a content ranging from 0.01% to 50% by weight, relative to the total weight of the composition, preferably ranging from 0.01% to 30% by weight.

The pulverulent dyestuffs may be chosen from pigments and nacres.

The pigments may be white or coloured, mineral and/or organic, and coated or uncoated. Among the mineral pigments which may be mentioned are titanium dioxide, optionally surface-treated, zirconium oxide, zinc oxide or cerium oxide, as well as iron oxide, chromium oxide, manganese violet, ultramarine blue, chromium hydrate and ferric blue. Among the organic pigments which may be mentioned are carbon black, pigments of D & C type, and lakes based on cochineal carmine or on barium, strontium, calcium or aluminium.

The nacres may be chosen from white nacreous pigments such as mica coated with titanium or with bismuth oxychloride, coloured nacreous pigments such as titanium mica with iron oxides, titanium mica with, in particular, ferric blue or chromium oxide, titanium mica with an organic pigment of the abovementioned type, and nacreous pigments based on bismuth oxychloride.

The liposoluble dyes are, for example, Sudan Red, D&C Red 17, D&C Green 6, β-carotene, soybean oil, Sudan Brown, D&C Yellow 11, D&C Violet 2, D&C Orange 5, quinoline yellow and annatto. The water-soluble dyes are, for example, beetroot juice and methylene blue.

The composition of the invention may also comprise any additive usually used in cosmetics, such as antioxidants, fillers, preserving agents, fragrances, neutralizing agents, thickeners, cosmetic or dermatological active agents such as, for example, emollients, moisturizers, vitamins and sunscreens, and mixtures thereof. These additives may be present in the composition in a content ranging from 0% to 20% (in particular from 0.01% to 20%) relative to the total weight of the composition and better still from 0.01% to 10% (if present).

Needless to say, a person skilled in the art will take care to select the optional additional additives and/or the amount thereof such that the advantageous properties of the composition according to the invention are not, or are not substantially, adversely affected by the addition envisaged.

The composition according to the invention may be manufactured by the known processes generally used in cosmetics or dermatology.

The invention is illustrated in greater detail in the examples which follow.

### Example 1:

A mascara having the composition below was prepared:
- Carnauba wax 2.6g
- Beeswax 3.3g
- Paraffin wax 10.4 g
- Hydrogenated jojoba oil 0.2g
- Hydrogenated palm oil 0.2 g
- Polyamide resin with ester end groups,
   sold under the name "Uniclear 100" by
   the company Arizona Chemical 1g
- 2-Amino-2-methyl-1,3-propanediol 0.8 g
- Triethanolamine 2.4 g
- Stearic acid 6.6 g
- Hydroxyethylcellulose 0.8 g
- Gum arabic 0.6 g
- Ethyl acrylate/methyl methacrylate
   copolymer (80/20) as an aqueous
   dispersion containing 50% AM
   (Daitosol 5000 AD from Saito) 5 g AM
- Polyamide fibres (3 mm long and 0.9 Dtex,
   from the company Paul Bonte) 1 g
- Black iron oxide 5 g
- Preserving agents qs
- Water qs 100 g

This mascara is easy to apply and adheres well to the eyelashes during and after application; the eyelashes are made up quickly.

The make-up result obtained gives the eyelashes a lengthened effect.

### Example 2:

A mascara having the composition below was prepared:
- Carnauba wax 2.6 g
- Beeswax 3.3 g
- Paraffin wax 10.4 g
- Hydrogenated jojoba oil 0.2 q
- Hydrogenated palm oil 0.2 g
- Polyamide resin sold under the name
   "Uni-Rez^{®} 126" by the company
   Arizona Chemical 1 g
- 2-Amino-2-methyl-1,3-propanediol 0.8 g
- Triethanolamine 2.4 g
- Stearic acid 6.6 g
- Hydroxyethylcellulose 0.8 g
- Gum arabic 0.6g
- Ethyl acrylate/methyl methacrylate
   copolymer (80/20) as an aqueous
   dispersion containing 50% AM
   (Daitosol 5000 AD from Saito) 5 g AM
- Polyamide fibres (3 mm long and 0.9 Dtex,
   from the company Paul Bonte) 1 g
- Black iron oxide 5 g
- Preserving agents qs
- Water qs 100 g

This mascara adheres well to the eyelashes during application and allows the eyelashes to be made up quickly.

## Claims

1. Composition comprising, in a. physiologically acceptable medium comprising a fatty phase, at least one first polyamide polymer with a weight-average molecular mass of less than 100 000, comprising a) a polymer skeleton containing amide repeating units, and b) optionally at least one pendent fatty chain and/or at least one terminal fatty chain, which may be functionalized, containing from 6 to 120 carbon atoms and being linked to these amide units, and one or more fibres, said polyamide polymer being chosen from polymers of formula (I) below, and mixtures thereof: 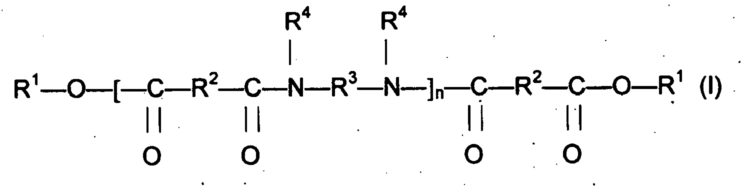 in which n denotes a number of amide units such that the number of ester groups represents from 10% to 50% of the total number of ester and amide groups; R¹ is, independently in each case, an alkyl or alkenyl group containing at least 4 carbon atoms; R² represents, independently in each case, a C₄ to C₄₂ hydrocarbon-based group, on condition that 50% of the groups R² represent a C₃₀ to C₄₂ hydrocarbon-based group; R³ represents, independently in each case, an organic group containing at least 2 carbon atoms, hydrogen atoms and optionally one or' more oxygen or nitrogen atoms; and R⁴ represents, independently in each case, a hydrogen atom, a C₁ to C₁₀ alkyl group or a direct bond to R³ or to another R⁴, such that the nitrogen atom to which R³ and R⁴ are both attached forms part of a heterocyclic structure defined by R⁴-N-R³, with at least 50% of the groups R⁴ representing a hydrogen atom.

2. Composition according to claim 1, **characterized in that** the fatty chains represent from 40% to 98% of the total number of amide units and of fatty chains.

3. Composition according to one of the preceding claims, **characterized in that** the average molar mass of the first polymer ranges from 1 000 to 100 000, preferably from 1 000 to 50 000 and better still from 1 000 to 30 000.

4. Composition according to one of the preceding claims, **characterized in that** the fatty chain(s) contain(s) from 12 to 68 carbon atoms.

5. Composition according to any one of the preceding claims, **characterized in that** R¹ is a C₁₂ to C₂₂ alkyl group.

6. Composition according to any one of Claims 1 to 5, **characterized in that** R² are groups containing from 30 to 42 carbon atoms.

7. Composition according to any one of Claims 1 to 6, **characterized in that** the polyamide polymer is a mixture of copolymers of a C₃₆ diacid condensed with ethylenediamine, having a weight-average molecular mass of about 6 000.

8. Composition according to one of the preceding claims, **characterized in that** the polyamide polymer is present in a content ranging from 0.01% to 10% by weight, relative to the total weight of the composition, preferably ranging from 0.05% to 5% by weight and better still ranging from 0.1% to 3% by weight.

9. Composition according to one of the preceding claims, **characterized in that** the fibre(s) is(are) chosen from silk, cotton, wool or flax fibres, cellulose fibres extracted in particular from wood, plants or algae, polyamide, cork, sugar can, rayon or viscose fibres, acetate fibres, in particular rayon acetate, cellulose acetate or silk acetate fibres, poly-(p-phenyleneterephthalamide) fibres, acrylic polymer fibres, in particular polymethyl methacrylate or poly-2-hydroxyethyl methacrylate fibres, polyolefin fibres and in particular polyethylene or polypropylene fibres, glass, silica or carbon fibres, in particular in graphite form, polytetrafluoroethylene, insoluble collagen, polyester, polyvinyl chloride or polyvinylidene chloride, polyvinyl alcohol, polyacrylonitrile, chitosan, polyurethane or polyethylene phthalate fibres, fibres formed from mixtures of polymers, and surgical fibres, and mixtures thereof.

10. Composition according to any one of the preceding claims, **characterized in that** the fibres are fibres of synthetic origin.

11. Composition according to one of the preceding claims, **characterized in that** the fibre(s) contain(s) a chemical group of the same chemical nature as that of the units of the structuring polymer or a group capable of forming physical bonds of the same type as that of the units of the polymer.

12. Composition according to any one of the preceding claims, **characterized in that** the fibres are polyamide fibres or poly-(p-phenyleneterephthamide) fibres.

13. Composition according to any one of the preceding claims, **characterized in that** the fibres have a length L and a diameter D such that L/D is chosen in the range from 1.5 to 2 500, preferably from 3.5 to 500 and better still from 5 to 150.

14. Composition according to one of the preceding claims, **characterized in that** the fibre is present in a content ranging from 0.1% to 40% by weight, relative to the total weight of the composition, preferably from 1% to 30% by weight and better still from 5% to 20% by weight.

15. Composition according to any one of the preceding claims, **characterized in that** it contains at least one wax.

16. Composition according to any one of the preceding claims, **characterized in that** it contains a volatile oil or organic solvent.

17. Composition according to one of the preceding claims, **characterized in that** it comprises a non-volatile oil.

18. Composition according to one of the preceding claims, **characterized in that** the fatty phase is present in a content ranging from 2% to 98% by weight, relative to the total weight of the composition, preferably ranging from 5% to 85% by weight.

19. Composition according to any one of the preceding claims, **characterized in that** it comprises an aqueous phase.

20. Composition according to any one of the preceding claims, **characterized in that** it comprises a second film-forming polymer which is different from the polyamide polymer.

21. Composition according to Claim 20, **characterized in that** the second film-forming polymer is chosen from the group formed by vinyl polymers, polyurethanes, polyesters, polyamides, polyureas and cellulose polymers.

22. Composition according to one of the preceding claims, **characterized in that** it also contains at least one dyestuff.

23. Composition according to one of the preceding claims, **characterized in that** it constitutes a care composition or make-up composition for keratin materials.

24. Composition according to one of the preceding claims, **characterized in that** it is in the form of a mascara, an eyeliner, a product for the eyebrows, a product for the lips, a face powder, an eyeshadow, a foundation, a make-up product for the body, a concealer product, a nail varnish, a skincare product or a haircare product.

25. Mascara comprising a composition according to any one of Claims 1 to 23.

26. Cosmetic process for making up or caring for the keratin materials of human beings, comprising the application of a cosmetic composition in accordance with one of Claims 1 to 24 to the keratin materials.

27. Use of a composition according to any one of Claims 1 to 24 to obtain a deposit which adheres to keratin materials.

28. Use of a mascara according to Claim 25 to thicken and/or lengthen the eyelashes.

## Patentansprüche

1. Zusammensetzung, die in einem physiologisch akzeptablen, eine Fettphase umfassenden Medium mindestens ein erstes Polyamidpolymer mit einer gewichtsmittleren Molmasse unter 100.000, das a) ein Polymergerüst, das Amid-Wiederholungseinheiten enthält, und b) gegebenenfalls mindestens eine als Seitenkette vorliegende Fettkette und/oder mindestens eine endständige Fettkette aufweist, die gegebenenfalls funktionalisiert sein können und 6 bis 120 Kohlenstoffatome aufweisen und an die Amideinheiten gebunden sind, und eine oder mehrere Fasern enthält, wobei das Polyamidpolymer unter den Polymeren der folgenden Formel (I) und deren Gemischen ausgewählt ist: 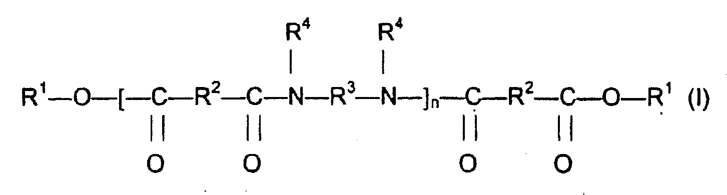 worin n die Zahl der Amideinheiten angibt, die so gewählt ist, dass die Anzahl der Estergruppen 10 bis 50 % der Gesamtzahl der Estergruppen und Amidgruppen ausmacht; die Gruppen R¹ jeweils unabhängig eine Alkyl- oder Alkenylgruppe mit mindestens 4 Kohlenstoffatomen bedeuten; die Gruppen R² jeweils unabhängig eine Kohlenwasserstoffgruppe mit 4 bis 42 Kohlenstoffatomen bedeuten, mit der Maßgabe, dass 50 % der Gruppen R² eine Kohlenwasserstoffgruppe mit 30 bis 42 Kohlenstoffatomen bedeuten; die Gruppen R³ jeweils unabhängig eine organische Gruppe bedeuten, die mindestens 2 Kohlenstoffatome, Wasserstoffatome und gegebenenfalls ein oder mehrere Sauerstoff- oder Stickstoffatome aufweist; und die Gruppen R⁴ jeweils unabhängig ein Wasserstoffatom, eine C₁₋₁₀-Alkylgruppe oder eine direkte Bindung zu R³ oder einer anderen Gruppe R⁴ bedeuten, so dass das Stickstoffatom, an das die Gruppen R³ und R⁴ beide gebunden sind, Teil einer heterocyclischen Struktur R⁴-N-R³ ist, wobei mindestens 50 % der Gruppen R⁴ ein Wasserstoffatom bedeuten.

2. Zusammensetzung nach Anspruch 1, **dadurch gekennzeichnet, dass** die Fettketten 40 bis 98 % der Gesamtzahl der Amideinheiten und Fettketten ausmachen.

3. Zusammensetzung nach einem der vorhergehenden Ansprüche, **dadurch gekennzeichnet, dass** die mittlere Molmasse des ersten Polymers im Bereich von 1.000 bis 100.000, vorzugsweise 1.000 bis 50.000 und noch besser 1.000 bis 30.000 liegt.

4. Zusammensetzung nach einem der vorhergehenden Ansprüche, **dadurch gekennzeichnet, dass** die Fettkette(n) 12 bis 68 Kohlenstoffatome aufweisen.

5. Zusammensetzung nach einem der vorhergehenden Ansprüche, **dadurch gekennzeichnet, dass** R¹ eine C₁₂₋₂₂-Alkylgruppe ist.

6. Zusammensetzung nach einem der Ansprüche 1 bis 5, **dadurch gekennzeichnet, dass** die Gruppen R² Gruppen mit 30 bis 42 Kohlenstoffatomen sind.

7. Zusammensetzung nach einem der Ansprüche 1 bis 6, **dadurch gekennzeichnet, dass** es sich bei dem Polyamidpolymer um ein Gemisch von Copolymeren einer mit Ethylendiamin kondensierten C₃₆-Disäure mit einer gewichtsmittleren Molmasse von etwa 6.000 handelt.

8. Zusammensetzung nach einem der vorhergehenden Ansprüche, **dadurch gekennzeichnet, dass** das Polyamidpolymer in einer Menge von 0,01 bis 10 Gew.-%, bezogen auf das Gesamtgewicht der Zusammensetzung, vorzugsweise in einer Menge von 0,05 bis 5 Gew.-% und noch besser in einer Menge von 0,1 bis 3 Gew.-% enthalten ist.

9. Zusammensetzung nach einem der vorhergehenden Ansprüche, **dadurch gekennzeichnet, dass** die Faser(n) unter den Seidenfasern, Baumwollfasern, Wollfasern, Leinenfasern, Cellulosefasern, die insbesondere aus Holz, Pflanzen und Algen gewonnen wurden, Polyamidfasern, Korkfasern, Zuckerrohrfasern, Rayonfasern oder Viskosefasern, Acetatfasern und insbesondere Fasern aus Rayonacetat, Celluloseacetat oder Seidenacetat, Poly-(*p*-phenylenterephthalamid)fasern, Acrylfasern und insbesondere Fasern aus Polymethylmethacrylat oder Poly-2-hydroxyethylmethacrylat, Polyolefinfasern und insbesondere Fasern aus Polyethylen oder Polypropylen, Glasfasern, Siliciumdioxidfasern, Kohlenstofffasern, insbesondere Fasern aus in Form von Graphit vorliegendem Kohlenstoff, Polytetrafluorethylenfasern, Fasern aus unlöslichem Collagen, Polyesterfasern, Polyvinylchloridfasern, Polyvinylidenchloridfasern, Polyvinylalkoholfasern, Polyacrylnitrilfasern, Chitosanfasern, Polyurethanfasern, Polyethylenphthalatfasern, Fasern aus Polymerengemischen, chirurgischen Fasern und deren Gemischen ausgewählt ist (sind).

10. Zusammensetzung nach einem der vorhergehenden Ansprüche, **dadurch gekennzeichnet, dass** es sich bei den Fasern um Fasern synthetischer Herkunft handelt.

11. Zusammensetzung nach einem der vorhergehenden Ansprüche, **dadurch gekennzeichnet, dass** die Faser(n) eine chemische Gruppe, die die gleiche chemische Natur hat wie die Einheiten des strukturierenden Polymers, oder eine Gruppe enthält (enthalten), die befähigt ist, physikalische Bindungen der gleichen Art wie die Einheiten des Polymers einzugehen.

12. Zusammensetzung nach einem der vorhergehenden Ansprüche, **dadurch gekennzeichnet, dass** es sich bei den Fasern um Polyamidfasern oder Poly-(*p*-phenylenterephthalamid)fasern handelt.

13. Zusammensetzung nach einem der vorhergehenden Ansprüche, **dadurch gekennzeichnet, dass** die Fasern eine Länge L und einen Durchmesser D aufweisen, die so sind, dass L/D im Bereich von 1,5 bis 2.500, vorzugsweise 3,5 bis 500 und besser 5 bis 150 liegt.

14. Zusammensetzung nach einem der vorhergehenden Ansprüche, **dadurch gekennzeichnet, dass** die Faser in einer Menge von 0,1 bis 40 Gew.-%, bezogen auf das Gesamtgewicht der Zusammensetzung, vorzugsweise 1 bis 30 Gew.-% und noch bevorzugter 5 bis 20 Gew.-% enthalten ist.

15. Zusammensetzung nach einem der vorhergehenden Ansprüche, **dadurch gekennzeichnet, dass** sie mindestens ein Wachs enthält.

16. Zusammensetzung nach einem der vorhergehenden Ansprüche, **dadurch gekennzeichnet, dass** sie ein flüchtiges Öl oder ein organisches Lösemittel enthält.

17. Zusammensetzung nach einem der vorhergehenden Ansprüche, **dadurch gekennzeichnet, dass** sie ein nichtflüchtiges Öl enthält.

18. Zusammensetzung nach einem der vorhergehenden Ansprüche, **dadurch gekennzeichnet, dass** die Fettphase in einem Mengenanteil von 2 bis 98 Gew.-%, bezogen auf das Gesamtgewicht der Zusammensetzung, und vorzugsweise 5 bis 85 Gew.-% enthalten ist.

19. Zusammensetzung nach einem der vorhergehenden Ansprüche, **dadurch gekennzeichnet, dass** sie eine wässrige Phase enthält.

20. Zusammensetzung nach einem der vorhergehenden Ansprüche, **dadurch gekennzeichnet, dass** sie ein zweites filmbildendes Polymer enthält, das von dem Polyamidpolymer verschieden ist.

21. Zusammensetzung nach Anspruch 20, **dadurch gekennzeichnet, dass** das zweite filmbildende Polymer unter den Vinylpolymeren, Polyurethanen, Polyestern, Polyamiden, Polyharnstoffen und Cellulosepolymeren ausgewählt ist.

22. Zusammensetzung nach einem der vorhergehenden Ansprüche, **dadurch gekennzeichnet, dass** sie ferner mindestens ein Farbmittel enthält.

23. Zusammensetzung nach einem der vorhergehenden Ansprüche, **dadurch gekennzeichnet, dass** es sich um eine Zusammensetzung für die Pflege oder zum Schminken von Keratinsubstanzen handelt.

24. Zusammensetzung nach einem der vorhergehenden Ansprüche, **dadurch gekennzeichnet, dass** sie als Mascara, Eyeliner, Produkt für die Augenbrauen, Produkt für die Lippen, Gesichtspuder, Lidschatten, Make-up, Make-up für den Körper, Abdeckprodukt, Nagellack, Hautpflegeprodukt oder Haarpflegeprodukt vorliegt.

25. Mascara, die eine Zusammensetzung nach einem der Ansprüche 1 bis 23 enthält.

26. Kosmetisches Verfahren für die Pflege oder zum Schminken menschlicher Keratinsubstanzen, das umfasst, eine Zusammensetzung nach einem der Ansprüche 1 bis 24 auf die Keratinsubstanzen aufzutragen.

27. Verwendung einer Zusammensetzung nach einem der Ansprüche 1 bis 24, um ein Depot zu bilden, das auf den Keratinsubstanzen haftet.

28. Verwendung einer Mascara nach Anspruch 25, um die Wimpern zu verdicken und/oder zu verlängern.

## Revendications

1. Composition comprenant, dans un milieu physiologiquement acceptable comprenant une phase grasse, au moins un premier polymère de polyamide de masse moléculaire moyenne en poids inférieure à 100 000, comportant a) un squelette polymérique ayant des motifs répétitifs amide, et b) éventuellement au moins une chaîne grasse pendante et/ou au moins une chaîne grasse terminale éventuellement fonctionnalisées, ayant de 6 à 120 atomes de carbone et étant liées à ces motifs amide, et une ou plusieurs fibres, ledit polymère de polyamide étant choisi parmi les polymères de formule (I) suivante et leurs mélanges : 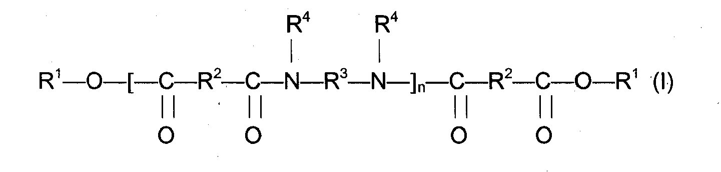 dans laquelle n désigne un nombre de motifs amide tel que le nombre de groupes ester représente de 10 % à 50 % du nombre total des groupes ester et amide ; R¹ est à chaque occurrence indépendamment un groupe alkyle ou alcényle ayant au moins 4 atomes de carbone ; R² représente à chaque occurrence indépendamment un groupe hydrocarboné en C₄ à C₄₂ à condition que 50 % des groupes R² représentent un groupe hydrocarboné en C₃₀ à C₄₂ ; R³ représente à chaque occurrence indépendamment un groupe organique pourvus d'au moins 2 atomes de carbone, d'atomes d'hydrogène et optionnellement d'un ou plusieurs atomes d'oxygène ou d'azote ; et R⁴ représente à chaque occurrence indépendamment un atome d'hydrogène, un groupe alkyle en C₁ à C₁₀ ou une liaison directe à R³ ou un autre R⁴ de sorte que l'atome d'azote auquel sont liés à la fois R³ et R⁴ fasse partie d'une structure hétérocyclique définie par R⁴-N-R³, avec au moins 50 % des R⁴ représentant un atome d'hydrogène.

2. Composition selon la revendication 1, **caractérisée par le fait que** les chaînes grasses représentent de 40 à 98 % du nombre total des motifs amide et des chaînes grasses.

3. Composition selon l'une des revendications précédentes, **caractérisée par le fait que** la masse molaire moyenne du premier polymère va de 1000 à 100 000, de préférence de 1000 à 50 000, et mieux de 1 000 à 30 000.

4. Composition selon l'une des revendications précédentes, **caractérisée par le fait que** la ou les chaînes grasses ont de 12 à 68 atomes de carbone.

5. Composition selon l'une des revendications précédentes, **caractérisée par le fait que** R¹ est un groupe alkyle en C₁₂ à C₂₂.

6. Composition selon l'une des revendications 1 à 5, **caractérisée par le fait que** R² sont des groupes ayant de 30 à 42 atomes de carbone.

7. Composition selon l'une des revendications 1 à 6, **caractérisée par le fait que** le polymère de polyamide est un mélange de copolymères d'un diacide en C₃₆ condensé sur l'éthylène diamine, de masse moléculaire moyenne en poids d'environ 6000.

8. Composition selon l'une des revendications précédentes, **caractérisée par le fait que** le polymère de polyamide est présent en une teneur allant de 0,01 % à 10 % en poids, par rapport au poids total de la composition, de préférence allant de 0,05 % à 5 % en poids, et mieux allant de 0,1 % à 3 % en poids.

9. Composition selon l'une des revendications précédentes, **caractérisée par le fait que** la ou les fibres sont choisies parmi les fibres de soie, de coton, de laine, de lin, de cellulose extraites notamment du bois, des légumes ou des algues, de polyamide, de liège, de canne à sucre, de rayonne, de viscose, d'acétate notamment d'acétate de rayonne, de cellulose ou de soie, les fibres de poly-(p-phénylène téréphtalamide), de polymère acrylique notamment de polyméthacrylate de méthyle ou de poly méthacrylate de 2-hydroxyéthyle, de polyoléfine notamment de polyéthylène ou de polypropylène, de verre, de silice, de carbone notamment sous forme graphite, de polytétrafluoroéthylène, de collagène insoluble, de polyesters, de polychlorure de vinyle ou de vinylidène, d'alcool polyvinylique, de polyacrylonitrile, de chitosane, de polyuréthane, de polyéthylène phtalate, les fibres de mélanges de polymères, les fibres chirurgicales et leurs mélanges.

10. Composition selon l'une quelconque des revendications précédentes, **caractérisée par le fait que** les fibres sont des fibres d'origine synthétique.

11. Composition selon l'une des revendications précédentes, **caractérisée par le fait que** la ou les fibres présentent un groupement chimique de même nature chimique que celle des motifs du polymère structurant ou un groupement capable de faire des liaisons physiques du même type que celui des motifs du polymère.

12. Composition selon l'une quelconque des revendications précédentes, **caractérisée par le fait que** les fibres sont des fibres de polyamide ou de poly-(p-phénylène téréphtalamide).

13. Composition selon l'une quelconque des revendications précédentes, **caractérisée par le fait que** les fibres ont une longueur L et un diamètre D tel que L/D est choisi dans la gamme allant de 1,5 à 2 500, de préférence de 3,5 à 500 et mieux de 5 à 150.

14. Composition selon l'une des revendications précédentes, **caractérisée par le fait que** la fibre est présente en une teneur allant de 0,1 à 40 % en poids, par rapport au poids total de la composition, de préférence de 1 à 30 % en poids, et mieux de 5 à 20 % en poids.

15. Composition selon l'une quelconque des revendications précédentes, **caractérisée par le fait qu'**elle contient au moins une cire.

16. Composition selon l'une quelconque des revendications précédentes, **caractérisée par le fait qu'**elle contient une huile ou un solvant organique volatile.

17. Composition selon l'une des revendications précédentes, **caractérisée par le fait qu'**elle comprend une huile non volatile.

18. Composition selon l'une des revendications précédentes, **caractérisée par le fait que** la phase grasse est présente en une teneur allant de 2 % à 98 % en poids, par rapport au poids total de la composition, de préférence allant de 5 % à 85 % en poids.

19. Composition selon l'une quelconque des revendications précédentes, **caractérisée par le fait qu'**elle comprend une phase aqueuse.

20. Composition selon l'une quelconque des revendications précédentes, **caractérisée par le fait qu'**elle comprend un deuxième polymère filmogène différent du polymère de polyamide.

21. Composition selon la revendication 20, **caractérisée par le fait que** le deuxième polymère filmogène est choisi dans le groupe formé par les polymères vinyliques, les polyuréthanes, les polyesters, les polyamides, les polyurées, les polymères cellulosiques.

22. Composition selon l'une des revendications précédentes, **caractérisée par le fait qu'**elle contient, en outre, au moins une matière colorante.

23. Composition selon l'une des revendications précédentes, **caractérisée par le fait qu'**elle constitue une composition de soin ou de maquillage des matières kératiniques.

24. Composition selon l'une des revendications précédentes, **caractérisée par le fait qu'**elle se présente sous forme de mascara, d'eye-liner, de produit pour les sourcils, de produit pour les lèvres, de fard à joues ou à paupières, de fond de teint, de produit de maquillage du corps, de produit anti-cernes, de vernis à ongles, de produit de soin de la peau, de produit pour les cheveux.

25. Mascara comprenant une composition selon l'une quelconque des revendications 1 à 23.

26. Procédé cosmétique de maquillage ou de soin des matières kératiniques des êtres humains, comprenant l'application d'une composition cosmétique conforme à l'une des revendications 1 à 24 sur les matières kératiniques.

27. Utilisation d'une composition selon l'une quelconque des revendications 1 à 24 pour l'obtention d'un dépôt adhérent sur les matières kératiniques.

28. Utilisation d'un mascara selon la revendication 25 pour épaissir et/ou allonger les cils.
